(19)

Europäisches Patentamt

European Patent Office

Office européen des brevets

(11) Publication number: **0 253 540**
**A1**

## (12) EUROPEAN PATENT APPLICATION

(21) Application number: 87305858.0

(22) Date of filing: 02.07.87

(51) Int. Cl.⁴: **C07C 29/15** , C07C 29/32 , C07C 31/02 , C10L 1/10 , B01J 27/051

(30) Priority: 15.07.86 GB 8617170

(43) Date of publication of application:
**20.01.88 Bulletin 88/03**

(84) Designated Contracting States:
**DE FR NL**

(71) Applicant: **Coal Industry (Patents) Limited**
**Hobart House Grosvenor Place**
**London SW1X 7AE(GB)**

(72) Inventor: **Gavin, Derek Gabriel**
**11 The Napping**
**Longhope Gloucestershire(GB)**
Inventor: **Richards, David Gareth**
**53 Roman Road**
**Cheltenham Gloucestershire(GB)**

(74) Representative: **Wood, John Irwin**
**Hobart House Grosvenor Place**
**London SW1X 7AE(GB)**

(54) **Synthesis gas conversion process.**

(57) A mixed alcohol product containing little or no methanol can be produced from synthesis gas by a process incorporating the synthesis of alcohols over a catalyst, the fractionation of the product to yield a desired mixed alcohol product and a fraction containing methanol, and recycling the methanol fraction to the synthesis reaction. Further production of methanol is inhibited. The product is suitable for blending with gasoline as an octane improver.

FIG. 1

## SYNTHESIS GAS CONVERSION PROCESS

This invention concerns a synthesis gas conversion process, more especially it concerns a process for converting synthesis gas into mixed alcohols substantially free of methanol.

The term "synthesis gas" as used herein is intended to encompass gases containing hydrogen and carbon monoxide from any source, including a conventional coal gasifier, heavy oil gasifier or from the partial oxidation of natural gas. Other means of generating synthesis gas include the steam reforming of natural gas, LPG or naphtha or methanol decomposition, including in situ methanol decomposition as described in more detail below.

It is known to make mixed alcohols from synthesis gas, by passing the gas over a catalyst. A variety of catalysts have been disclosed, mainly based on iron oxide, copper with cobalt, rhodium or molybdenum. For example, the Synol process employed an iron oxide/potassium oxide catalyst to produce a mixture of alcohols (55%) and hydrocarbons (40%). The mixture boiled in the range of 34° to 360°C, making them unsuitable for blending into modern gasoline. More recent processes published in the patent literature have concentrated on promoted rhodium catalysts, mainly on silica supports. Rhodium is, however, extremely expensive and is not particularly selective for producing alcohols at high rates because low value hydrocarbons are simultaneously produced.

It is also known to use molybdenum sulphide as a catalyst for making liquid hydrocarbons and organic oxygenates. For example, USP 2,490,488 discloses $MoS_2$ modified by the addition of alkali metal compounds. European Patent Application No.0119609 (Dow) discloses a number of catalysts, both supported and bulk catalysts, and Examples 9 and 18 relate to $MoS_2$ Promoted with potassium carbonate. Example 9 discloses 32% $MoS_2$ and 7.5% $K_2CO_3$ on active carbon and dried at 300°C in nitrogen yielding 121g of alcohols /h/kg catalyst at a selectivity of 91.6% ($CO_2$-free basis). Example 18 discloses a clay-supported catalyst containing 66% $MoS_2$ and 10% $K_2CO_3$ which yields 367g of alcohols /h/kg catalyst at 87% selectivity.

Another recent European Application, No 0 149 255 (Union Carbide), also discloses the production of alcohols using alkali-promoted $MoS_2$. The best results were disclosed in Examples 8 and 21, both giving a yield of 256g/h/kg at 80% selectivity using unsupported $MoS_2$ with cesium promoters. A mixture of unsupported $MoS_2$ and KOH gave 221g/h/kg at 80% selectivity.

USP 4,243,553 discloses the decomposition of ammonium thiomolydbate to yield high surface area $MoS_2$ for use in water gas shift and methanation reactions. No mention is made of alcohols in this document, but it is noted that reaction temperatures are relatively high.

Our co-pending UK Patent Application No 2,185,907A describes an improved catalyst based on $MoS_2$ and $K_2CO_3$ on an active carbon carrier and which is capable under optimum conditions of giving high yields of mixed alcohols, eg up to 700g/h/kg catalyst with selectivities of from 85 to 95% alcohols by weight on a $CO_2$-free basis. The teaching of said Application is incorporated herein by reference.

It is now recognised that the use of lead alkyls as octane enhancers in gasoline are undesirable because of contamination of the environment by toxic lead compounds. Favoured alternatives to lead alkyls are oxygenated organic compounds such as mixed alcohols and methyl tertiary butyl ether.

Alcohols which are useful as antiknock agents are ethanol, propanol, butanol and pentanol, and mixtures thereof. Methanol improves octane ratings of gasoline but has well-known drawbacks including its toxicity, its ability to increase the vapour pressure of the gasoline and to reduce the distillation boiling points of lighter gasoline components, and its adverse effect on the tolerance of gasoline to water before phase separation occurs. The problem exhibited by methanol are of sufficient seriousness that legislation is being considered to limit its use to below 3% by wt of gasoline in Europe and in USA. It would be advantageous to make mixed alcohols from synthesis gas in one stage, containing no, or substantially no, methanol.

Unfortunately, all the known processes and catalysts yield mixed alcohols containing from 30 to 80% of methanol, depending upon conditions and the $H_2$: CO ratio. In addition, it is found that lower methanol contents, eg 30%, correspond to lower yields of total alcohols.

The present invention provides a process for the production of ethanol, propanol and butanol with substantially no net production of methanol from synthesis gas, comprising passing the synthesis gas over an alcohol synthesis catalyst under alcohol synthesis conditions, in the present of methanol and fractionating the product of alcohol synthesis to yield a fraction containing ethanol, propanol and butanol and a fraction containing methanol and recycling said fraction containing methanol, whereby there is substantially

2

no net production of methanol. The invention further provides a process for the production of mixed alcohols comprising a desired small quantity of methanol, comprising the modification of the above process by recycling a proportion of the methanol in the product of the alcohol synthesis and incorporating the remaining proportion in a mixed alcohol product containing the desired small quantity of methanol.

It has previously been suggested in alcohol synthesis processes to recycle unreacted synthesis gas. This is quite distinct from the recycle of methanol according to the present invention, and the process of the present invention may also include unreacted gas recycle.

The present invention is also distinct from methanol homologation reactions, in which methanol and synthesis gas are fed over a homogation catalyst in order to form the higher alcohol homologues, especially ethanol and propanol. Such processes are described, for example, in European Patent 84 833B and European Patent Application 13464A. Exemplary catalysts are based on cobalt and/or ruthenium. A variant of such processes is described in USP 3972 952, in which a methanol/ethanol mixture is reacted with synthesis gas over an alkali metal or alkaline earth metal catalyst supported on alumina. Conversions to higher alcohols were extremely low, however. Promoted cobalt catalysts tend to give high yields of by-products such as acetaldehyde, methyl acetate and/or hydrocarbons. In homologation reactions, methanol is a primary reactant, whereas in the process of the present invention, methanol is added to bring the reaction producing methanol closer to equilibrium, and hence to prevent methanol formation. Excess methanol will decompose to carbon monoxide and hydrogen. These reactions are discussed in more detail below.

It is believed that the chemical reactions taking place in the synthesis reactor in the present invention may be adequately summarised by the following equations

$CO + 2H_2 : CH_3OH$ (1)

$CH_3OH : CO + 2H_2$ (2)

$3CO + 3H_2 : C_2H_5OH + CO_2$ (3)

$5CO + 4H_2 : C_3H_7OH + 2CO_2$ (4)

$7CO + 5H_2 : C_4H_9OH + 3CO_2$ (5)

$CO + H_2O : H_2 + CO_2$ (6)

$2CO + 2H_2 : CH4 + CO_2$ (7)

$4CO + 3H_2 : C_2H_6 + 2CO_2$ (8)

Reactions (3) to (8) are reversible but the equilibria lie to the right under the conditions preferably used in the present invention. Water is produced as a by-product in reactions (3) to (5), (7) and (8), but it is nearly fully reacted and removed in the water gas shift reaction (6) over the $MoS_2$ catalyst. Reactions (7) and (8) producing hydrocarbon gases are minor reactions at temperatures of 260 to 350°C, but become significant at higher temperatures. Reactions (1) and (2) are reversible and can be at equilibrium in short residence times at certain temperatures over, for example, the activated $MoS_2$ catalyst of our aforementioned Application.

The supply of methanol to the synthesis reactor according to the present invention has the effect that reaction (1) producing methanol can be halted or reversed. It has also been discovered that the preferred catalyst according to our aforementioned Application is capable of promoting a constant mass change of methanol, higher alcohols and water, such that if the mass of methanol which is produced or decomposed is lowered, then more ethanol and propanol is made at the same temperature to maintain the overall change of alcohols and water constant in mass units. Hence, by adding methanol to the reactor, the quantity of methanol produced is reduced and the quantity of higher alcohols is increased.

If more methanol than that determined by chemical equilibrium is added, then methanol decomposes by reaction (2) to synthesis gas. It is therefore not essential that synthesis gas as such should form part of the feedstock of the process of the present invention, and the feedstock may be methanol, methanol and hydrogen, methanol and carbon monoxide as well as methanol with synthesis gas.

It is preferred to operate the process of the invention using methanol with synthesis gas. Although there is no significance to the source of the methanol, it is preferably recycled methanol from the fractionation of the product from the synthesis reactor. The recycle methanol may be supplemented with methanol imported to the process from another source.

When synthesis gas is used as part of the feedstock, the $H_2: CO$ volume ratio is suitably in the range from 0.3 to 3:1. The $H_2:CO$ ratio influences the composition of the alcohols from the synthesis reactor, the product yields per unit time and the ratio of $H_2$ to CO consumed. In "once-through" experiments, a feed ratio of 2:1 $H_2:CO$ gave a ratio of $H_2:CO$ consumed of 1.5 to 1 whereas a feed ratio of 1:1 $H_2:CO$ gave a ratio of $H_2:CO$ consumer of 1.2:1. The 2:1 feedstock gave 80% methanol in the products whereas the 1:1 feedstock yielded products containing 50% methanol. At lower ratios, eg. 0.3:1 $H_2:CO$, the product yields deteriorated. Preferred $H_2:CO$ ratios are approximately 1.2:1 when no methanol supplement is incorporated

3

but all the methanol made is recycled. If supplemental methanol is used, a lower $H_2$:CO ratio, eg about 0.9:1, would be desirable since methanol provides $H_2$ and CO at a ratio of 2:1. Suitably, the methanol to carbon monoxide volume ratio is from 1:5 to 1:0, and suitably the methanol to hydrogen ratio is from 1:0 to 1:1

Carbon dioxide may be present in the feedstock up to about 30% by volume, but is found to reduce the yield of alcohols, probably because of dilution of reactants. For instance at 0% $CO_2$, the yield of alcohols was 250g/h/kg catalyst, whereas at 30% $CO_2$ in the synthesis gas and at the same reactor pressure of 100 bar, the yield was 125g/h/kg calalyst. The feedstock may contain other components which do not significantly adversely affect the reactions of the catalyst. For example, the preferred catalyst system is resistant to poisoning by $H_2S$, and hence a feedstock may comprise $H_2S$, which may be advantageous to maintain the catalyst sulphided.

Suitable catalysts for use in the present invention include those known in the art as alcohol synthesis catalysts, such as iron oxide, copper with cobalt, and rhodium or molybdenum sulphide promoted with an alkali metal, eg promoted with potassium carbonate. Our UK Application No 86 02654 describes highly active catalysts based on molybdenum sulphide absorbed on to particulate active carbon and promoted by potassium carbonate. Catalysts based on this formulation are preferred for use in the present invention for the following reasons:

    a) Mixed alcohol yields of 680g/h/kg catalyst have been found under suitable conditions;

    b) High selectivities of alcohols compared to $CH_4$/$C_2H_6$ were found, for example in the range 83 to 95 wt% ($CO_2$ free basis);

    c) the catalyst has a high water gas shift capacity so that most of the excess combined oxygen is eliminated as $CO_2$ instead of as $H_2O$, leading to an essentially dry alcohol mixture;

    d) The catalyst is highly active for equilibrating synthesis gas with methanol.

The process is preferably carried out at temperature in the range 260 to 350°C, especially 270 to 300°C, and preferably at a pressure of at least 50 bar, especially 100 to 160 bar. Preferred GHSV's are from 1,000 to 50,000 $h^{-1}$, especially 2,000 - 20,000 $h^{-1}$. In general, the alcohols yield improves with increasing GHSV while gas make is slightly reduced. The preferred catalyst may exhibit a falling off of activity during an initial period; this can be compensated for by raising the temperature slightly, eg in 5°C steps, until a suitable activity is obtained.

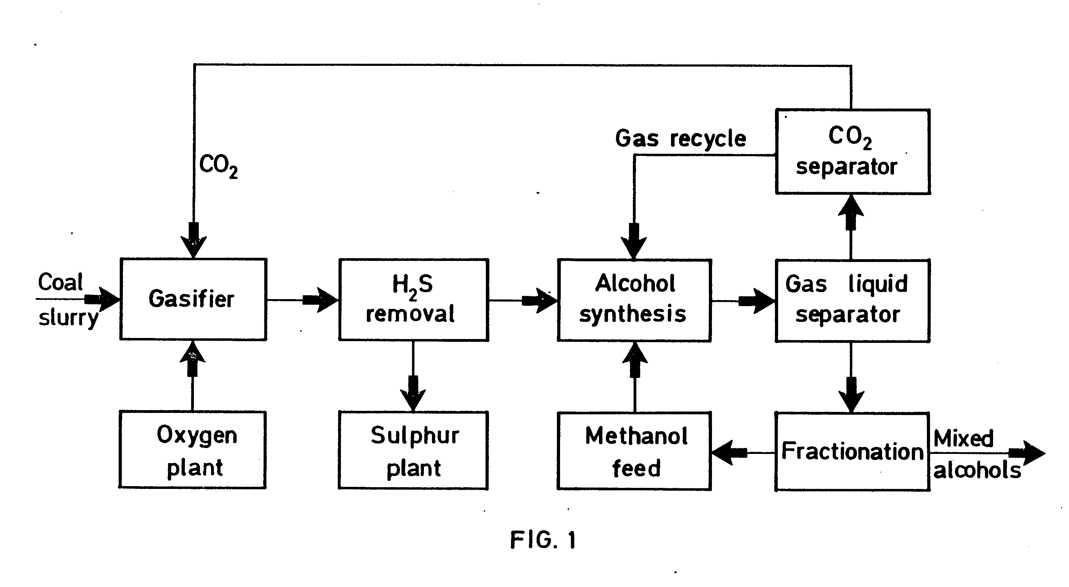

The invention may be more fully described with reference to the accompanying Fig 1, which is a Flow Diagram of one embodiment of a process according to the present invention.

Synthesis gas is produced in a coal gasifier, and is passed to a synthesis reactor after removal of the majority of the $H_2S$ in the product synthesis gas. The synthesis reactor can be one of a variety of designs, eg. a tubular or squat fixed bed, moving bed or fluidised bed, or an inert liquid recycling reactor. Because of the nature of the process, some care needs to be taken in the reactor design to ensure adequate heat removal and temperature control, but this lies within the expertise of the competent chemical engineer. Make up and recycled synthesis gas are co-fed with methanol into the reactor. The products from the synthesis reactor are passed to a gas-liquid separator; the resulting gas stream is further separated into $CO_2$ which is optionally recycled to the gasifier or may be discarded, hydrocarbon gases in small amount which are removed in a bleed gas (not shown) and unreacted synthesis gas which is recycled to the synthesis reactor. The liquid stream from the gas-liquid separator is passed to a fractionating column from which all the methanol produced is recycled to the synthesis reactor and a mixed alcohol product containing ethanol, propanol and butanol is produced, which may contain small quantities of water. The mixed alcohol product contains approximately 80% by wt of ethanol and 20% of propanol/butanol.

The process of the invention has the advantage that the elimination of a net make of methanol has benefits in the energy balance of the synthesis. Methanol synthesis is significantly exothermic and if this heat is not liberated, then more heat from the other reactions can be accommodated for the same heat duty, or temperature rise in the catalyst bed.

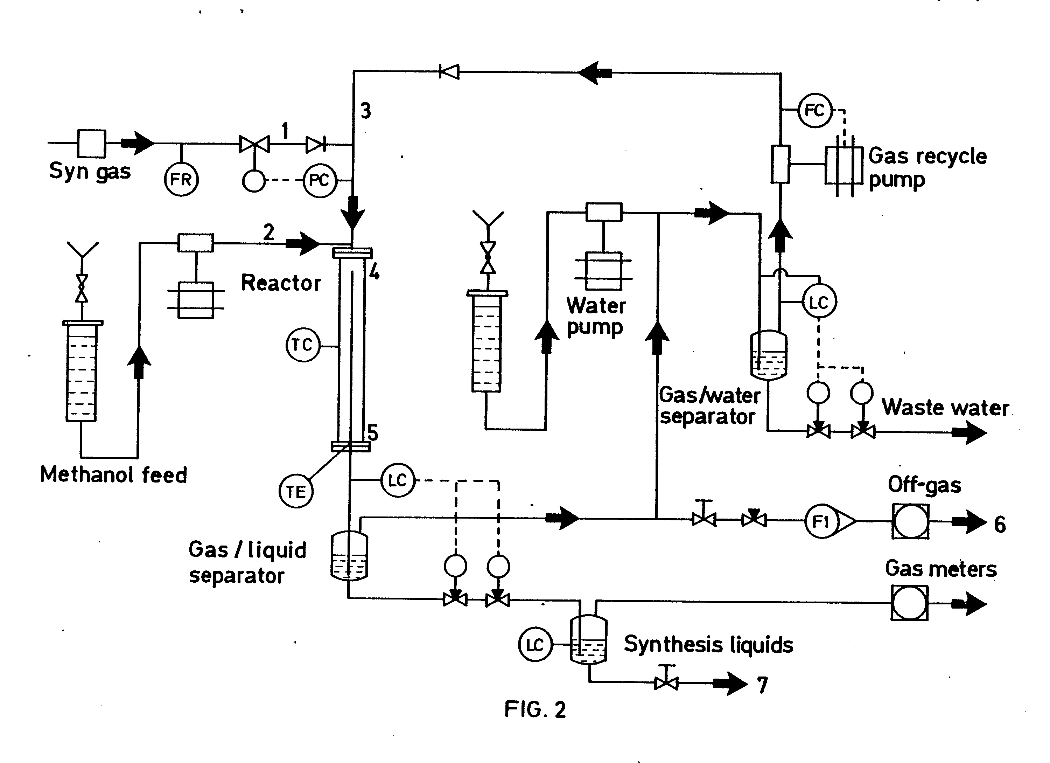

The invention will now be described by way of example only. The principle of the process was proved in tests carried out in an experimental unit shown in Fig 2.

Synthesis gas at a $H_2$:CO ratio of 1:1 is fed to a fixed bed reactor containing 150ml of catalyst. Methanol was pumped from a storage vessel at four different rates to the reactor. The reactor was also fed with recycle gas. The reactor was held at 300°C and a pressure of 100 bar with a feedstock throughput (gas hourly space velocity) of 2,700 $h^{-1}$. The products of reaction were cooled to ambient temperature in the gas/liquid separator, and the liquid products were collected, weighed and analysed. The gaseous products were split, with 50% being taken as product, being depressurised, measured and analysed, and the remainder being recycled to the reactor. Because the gas made contained less than 5% $CO_2$ by vol, the water scrubbing section shown in the figure was dispensed with.

The catalyst used had a loading of 21% $MoS_2$ and 6% $K_2CO_3$ on a coconut shell active carbon carrier, and was prepared as described in Example 2 of our UK Application No 83 02654.

The effects of feeding methanol in various quantities in the process were determined by analysing and calculating the composition of the various streams at points 1 to 7 shown on Figure 2.

## Example 1.

The reactor was run with zero methanol feed. The results of the test are shown in Tables 1 and 5. The overall yields of alcohols can be assessed from the stream 7 by subtracting the methanol feed value in stream 2. The product yields and composition of the alcohol which are summarised in Table 5 were 183g/h/kg catalyst of mixed alcohols containing 77% methanol and 23% other alcohols.

## Example 2

The reactor was run with 171g of methanol/h/kg catalyst. The results are shown in Tables 2 and 5. There was a net methanol make leading to a composition of 39% methanol and 61% higher alcohols at a yield of 170 g of alcohols/h/kg catalyst.

## Example 3

The reactor was run with 286g of methanol/h/kg catalyst. The results are shown in Tables 3 and 5. In this case there was a net decomposition of methanol of 41g. If the mode of operation were to continue, there would need to be a net import of methanol of 41g/h. The yield of alcohols was 129g/h/kg catalyst at 0% methanol, 78% ethanol and 22% propanol/butanol.

## Example 4

The reactor was run with 402g of methanol/h/kg catalyst. results are shown in Tables 4 and 5. There was a net loss of methanol of 48g requiring a net import of 48g of methanol. Yield was 123g alcohols/h/kg catalyst at 79% ethanol/21% propanol and butanol.

In examples 2 and 3, the methanol leaving the reactor was at 238 and 244 g/h/kg catalyst respectively; in the first case methanol was made and in the second case methanol was decomposed. This indicates that the reaction between methanol, hydrogen and carbon monoxide is at thermodynamic equilibrium. Calculations of the thermodynamic equilibria of the composition for examples 2 and 3 indicate that the reactor temperature should have been at 350 to 360°C whereas the average recorded temperatures were 315 to 330°C. We believe this was caused by localised hot spots in the experimental fixed bed reactor which by the nature of its design had a poor heat transfer.

## Examples 5 & 6

In these examples, a more active catalyst was used, again based on $MoS_2/K_2CO_3$ on carbon. The $H_2$:CO ratio was 2:1 and no methanol was fed to the reactor. The examples are reported to indicate that high yields of higher alcohols are possible in the process of the invention.

The results shown in Table 6 show yields of alcohol of 452 and 682g/h/kg catalyst at 54% and 66% methanol respectively. If the methanol in the product was recycled then there is the possibility of attaining the high yields of 682g/h/kg catalyst at zero methanol provided that, as the methanol yield decreased to zero then the yield of higher alcohols increased to maintain an overall constant yield. This effect occurred in examples 1 to 4.

Table 1 Page 1      Alcohols Synthesis    Mass Balance for Example 1
                    RUN CM11

```
W.B.number                10A
Catalyst vol. (l)         0.150
Catalyst wt.(kg)          0.083
Mass balance time(h)       1.0
Liquid analyses
   Wt.(g/h)               15.5
   MeOH (%)               75.0
   EtOH (%)               18.0
   PrOH (%)                5.0
   H2O (%)                 2.0
Off-gas
   Vol.(std.l/h)         256.0
   H2(%)                  48.1
   CO                     49.5
   CH4                     0.7
   C2H6                    0.1
   CO2                     1.6
Gas recycle
   Vol.(std.l/h)         150.0
Methanol IN (g/h)          0.0
Operating conditions
   Temp.(*C)    1          260
                2          287
                3          298
                4          297
                5          301
                6          300
Pressure (bar)            100
GHSV (1/h)               2707
CO conversion (g/h/k     278.6
CO conversion (%)          8.4
```

Synthesis Mass Balance (g/h/kg.cat)

Table 1,  Page 2

| Component | 1 Syn gas feed | 2 MeOH feed | 3 Gas recycle | 4 Reactor inlet | 5 Reactor outlet . | 6 Off-gas | 7 Synthesis liquids |
|---|---|---|---|---|---|---|---|
| H2 | 157.1 | − | 77.6 | 234.7 | 210.1 | 132.5 | − |
| CO | 2187.1 | − | 1118.2 | 3305.3 | 3026.7 | 1908.4 | − |
| CH4 | − | − | 9.0 | 9.0 | 24.5 | 15.4 | − |
| C2H6 | − | − | 2.4 | 2.4 | 6.6 | 4.1 | − |
| CO2 | − | − | 56.8 | 56.8 | 153.7 | 96.9 | − |
| CH3OH | − | 0.0 | − | 0.0 | 140.1 | − | 140.i |
| C2H5OH | − | − | − | − | 33.6 | − | 33.6 |
| C3H7OH | − | − | − | − | 9.3 | − | 9.3 |
| H2O | − | − | − | − | 3.7 | − | 3.7 |
| Totals | 2344.1 | 0.0 | 1264.1 | 3608.2 | 3608.2 | 2157.4 | 186.7 |

| | |
|---|---|
| 1+2+3 | 3608.2 |
| 6+7+3 | 3608.2 |
| 1+2 | 2344.1 |
| 6+7 | 2344.1 |

Synthesis Mass Balance (mols/h/kg.cat)

Table 1   Page 3

| Component | 1 Syn gas feed | 2 MeOH feed | 3 Gas recycle | 4 Reactor inlet | 5 Reactor outlet | 6 Off-gas | 7 Synthesis liquids |
|---|---|---|---|---|---|---|---|
| H2 | 78.5 | — | 38.8 | 117.3 | 105.0 | 66.2 | — |
| CO | 78.1 | — | 39.9 | 118.0 | 108.1 | 68.2 | — |
| CH4 | — | — | 0.6 | 0.6 | 1.5 | 1.0 | — |
| C2H6 | — | — | 0.1 | 0.1 | 0.2 | 0.1 | — |
| CO2 | — | — | 1.3 | 1.3 | 3.5 | 2.2 | — |
| CH3OH | — | 0.0 | — | 0.0 | 4.4 | — | 4.4 |
| C2H5OH | — | — | — | — | 0.7 | — | 0.7 |
| C3H7OH | — | — | — | — | 0.1 | — | 0.1 |
| H2O | — | — | — | — | 0.2 | — | 0.2 |
| Totals | 156.6 | 0.0 | 80.7 | 237.3 | 223.8 | 137.7 | 5.4 |

| 1+2+3 | 237.3 |
|---|---|
| 6+7+3 | 223.8 |

0 253 540

Table 2  Page 1        Alcohols Synthesis    Mass Balance for Example 2
                       RUN CM11

```
W.B.number                 8A
Catalyst vol. (1)          0.150
Catalyst wt.(kg)           0.083
Mass balance time(h)        5.0
Liquid analyses
   Wt.(g/h)                29.2
   MeOH (%)                67.5
   EtOH (%)                23.3
   PrOH (%)                 6.2
   H2O (%)                  3.0
Off-gas
   Vol.(std.l/h)          250.4
   H2(%)                   47.6
   CO                      46.3
   CH4                      2.0
   C2H6                     0.2
   CO2                      4.0
Gas recycle
   Vol.(std.l/h)          150.0
Methanol IN (g/h)          14.2
Operating conditions
   Temp.(*C)   1           265
               2           300
               3           320
               4           330
               5           330
               6           305
Pressure (bar)             100
GHSV (1/h)                2669
CO conversion (g/h/k      440.2
CO conversion (%)          13.6
```

Synthesis Mass Balance (g/h/kg.cat)

Table 2  Page 2

| Component | 1 Syn gas feed | 2 MeOH feed | 3 Gas recycle | 4 Reactor inlet | 5 Reactor outlet | 6 Off-gas | 7 Synthesis liquids |
|---|---|---|---|---|---|---|---|
| H2 | 157.0 | — | 76.8 | 233.8 | 205.0 | 128.2 | — |
| CO | 2186.2 | — | 1045.9 | 3232.1 | 2791.9 | 1746.0 | — |
| CH4 | — | — | 25.8 | 25.8 | 68.9 | 43.1 | — |
| C2H6 | — | — | 4.8 | 4.8 | 12.9 | 8.1 | — |
| CO2 | — | — | 142.0 | 142.0 | 379.0 | 237.0 | — |
| CH3OH | — | 171.1 | — | 171.1 | 237.5 | — | 237.5 |
| C2H5OH | — | — | — | — | 82.0 | — | 82.0 |
| C3H7OH | — | — | — | — | 21.8 | — | 21.8 |
| H2O | — | — | — | — | 10.6 | — | 10.6 |
| Totals | 2343.2 | 171.1 | 1295.4 | 3809.7 | 3809.7 | 2162.4 | 351.8 |

| | |
|---|---|
| 1+2+3 | 3809.7 |
| 6+7+3 | 3809.7 |
| 1+2 | 2514.3 |
| 6+7 | 2514.3 |

Synthesis Mass Balance (mols/h/kg.cat)

0 253 540

**Table 2   Page 3**

| Component | 1 Syn gas feed | 2 MeOH feed | 3 Gas recycle | 4 Reactor inlet | 5 Reactor outlet | 6 Off-gas | 7 Synthesis liquids |
|---|---|---|---|---|---|---|---|
| H2 | 78.5 | – | 38.4 | 116.9 | 102.5 | 64.1 | – |
| CO | 78.1 | – | 37.4 | 115.4 | 99.7 | 62.4 | – |
| CH4 | – | – | 1.6 | 1.6 | 4.3 | 2.7 | – |
| C2H6 | – | – | 0.2 | 0.2 | 0.4 | 0.3 | – |
| CO2 | – | – | 3.2 | 3.2 | 8.6 | 5.4 | – |
| CH3OH | – | 5.3 | – | 5.3 | 7.4 | – | 7.4 |
| C2H5OH | – | – | – | – | 1.8 | – | 1.8 |
| C3H7OH | – | – | – | – | 0.3 | – | 0.3 |
| H2O | – | – | – | – | 0.6 | – | 0.6 |
| Totals | 156.6 | 5.3 | 80.8 | 242.7 | 225.7 | 134.8 | 10.1 |

| 1+2+3 | 242.7 |
|---|---|
| 6+7+3 | 225.7 |

0 253 540

Table 3 Page 1 Alcohols Synthesis Mass Balance for Example 3
RUN CM11

```
W.B.number                    7A
Catalyst vol. (1)           0.150
Catalyst wt.(kg)            0.083
Mass balance time(h)          4.5
Liquid analyses
   Wt.(g/h)                  32.7
   MeOH (%)                  62.0
   EtOH (%)                  25.5
   PrOH (%)                   7.1
   H2O (%)                    5.3
Off-gas
   Vol.(std.1/h)            259.0
   H2(%)                     48.3
   CO                        47.7
   CH4                        1.3
   C2H6                       0.1
   CO2                        2.7
Gas recycle
   Vol.(std.1/h)            100.0
Methanol IN (g/h)            23.7
Operating conditions
   Temp.(*C)   1              270
               2              300
               3              315
               4              315
               5              310
               6              293
Pressure (bar)               100
GHSV (1/h)                   2393
CO conversion (g/h/k        287.0
CO conversion (%)            10.0
```

Synthesis Mass Balance (g/h/kg.cat)

Table 3  Page 2

| Component | 1<br>Syn gas<br>feed | 2<br>MeOH feed | 3<br>Gas recycle | 4<br>Reactor<br>inlet | 5<br>Reactor<br>outlet | 6<br>Off-gas | 7<br>Synthesis<br>liquids |
|---|---|---|---|---|---|---|---|
| H2 | 154.2 | — | 52.0 | 206.2 | 186.5 | 134.6 | — |
| CO | 2147.6 | — | 718.4 | 2866.0 | 2579.0 | 1860.6 | — |
| CH4 | — | — | . 11.2 | 11.2 | 40.2 | 29.0 | — |
| C2H6 | — | — | 1.6 | 1.6 | 5.8 | 4.2 | — |
| CO2 | — | — | 63.9 | 63.9 | 229.4 | 165.5 | — |
| CH3OH | — | 285.5 | — | 285.5 | 244.3 | — | 244.3 |
| C2H5OH | — | — | — | — | 100.5 | — | 100.5 |
| C3H7OH | — | — | — | — | 28.0 | — | 28.0 |
| H2O | — | — | — | — | 20.9 | — | 20.9 |
| Totals | 2301.9 | 285.5 | 847.0 | 3434.4 | 3434.4 | 2193.8 | 393.6 |

| | |
|---|---|
| 1+2+3 | 3434.4 |
| 6+7+3 | 3434.4 |
| 1+2 | 2587.4 |
| 6+7 | 2587.4 |

Synthesis Mass Balance (mols/h/kg.cat)

0 253 540

Table 3  Page 3

| Component | 1 Syn gas feed | 2 MeOH feed | 3 Gas recycle | 4 Reactor inlet | 5 Reactor outlet | 6 Off-gas | 7 Synthesis liquids |
|---|---|---|---|---|---|---|---|
| H2 | 77.1 | — | 26.0 | 103.1 | 93.3 | 67.3 | — |
| CO | 76.7 | — | 25.7 | 102.4 | 92.1 | 66.4 | — |
| CH4 | — | — | 0.7 | 0.7 | 2.5 | 1.8 | — |
| C2H6 | — | — | 0.1 | 0.1 | 0.2 | 0.1 | — |
| CO2 | — | — | 1.5 | 1.5 | 5.2 | 3.8 | — |
| CH3OH | — | 8.9 | — | 8.9 | 7.6 | — | 7.6 |
| C2H5OH | — | — | — | — | 2.2 | — | 2.2 |
| C3H7OH | — | — | — | — | 0.4 | — | 0.4 |
| H2O | — | — | — | — | 1.2 | — | 1.2 |
| Totals | 153.8 | 8.9 | 53.8 | 216.6 | 204.7 | 139.4 | 11.4 |

1+2+3    216.6
6+7+8    204.7

0 253 540

Table 4 Page 1          Alcohols Synthesis    Mass Balance for Example 4
                        RUN CM11

| | |
|---|---|
| N.B.number | 9A |
| Catalyst vol. (1) | 0.150 |
| Catalyst wt.(kg) | 0.083 |
| Mass balance time(h) | 5.0 |
| Liquid analyses | |
| Wt.(g/h) | 40.5 |
| MeOH (%) | 72.5 |
| EtOH (%) | 20.0 |
| PrOH (%) | 5.2 |
| H2O (%) | 2.3 |
| Off-gas | |
| Vol.(std.1/h) | 252.0 |
| H2(%) | 47.2 |
| CO | 45.5 |
| CH4 | 2.6 |
| C2H6 | 0.2 |
| CO2 | 4.5 |
| Gas recycle | |
| Vol.(std.1/h) | 150.0 |
| Methanol IN (g/h) | 33.4 |
| Operating conditions | |
| Temp.(*C)   1 | 255 |
| 2 | 280 |
| 3 | 292 |
| 4 | 300 |
| 5 | 305 |
| 6 | 295 |
| Pressure (bar) | 100 |
| GHSV (1/h) | 2680 |
| CO conversion (g/h/k | 394.1 |
| CO conversion (%) | 12.5 |

Synthesis Mass Balance (g/h/kg.cat)

Table 4    Page 2

| Component | 1 Syn gas feed | 2 MeOH feed | 3 Gas recycle | 4 Reactor inlet | 5 Reactor outlet | 6 Off-gas | 7 Synthesis liquids |
|---|---|---|---|---|---|---|---|
| H2 | 152.3 | – | 76.2 | 228.5 | 204.1 | 128.0 | – |
| CO | 2120.9 | – | 1027.9 | 3148.8 | 2754.7 | 1726.8 | – |
| CH4 | – | – | 33.6 | 33.6 | 89.9 | 56.4 | – |
| C2H6 | – | – | 4.8 | 4.8 | 13.0 | 8.1 | – |
| CO2 | – | – | 159.7 | 159.7 | 428.1 | 268.4 | – |
| CH3OH | – | 402.4 | – | 402.4 | 353.8 | – | 353.8 |
| C2H5OH | – | – | – | – | 97.6 | – | 97.6 |
| C3H7OH | – | – | – | – | 25.4 | – | 25.4 |
| H2O | – | – | – | – | 11.2 | – | 11.2 |
| Totals | 2273.2 | 402.4 | 1302.2 | 3977.8 | 3977.8 | 2187.7 | 488.0 |

| | |
|---|---|
| 1+2+3 | 3977.8 |
| 6+7+3 | 3977.8 |
| 1+2 | 2675.6 |
| 6+7 | 2675.6 |

Synthesis Mass Balance (mols/h/kg.cat)

0 253 540

Table 4   Page 3

| Component | 1<br>Syn gas<br>feed | 2<br>MeOH feed | 3<br>Gas recycle | 4<br>Reactor<br>inlet | 5<br>Reactor<br>outlet | 6<br>Off-gas | 7<br>Synthesis<br>liquids |
|---|---|---|---|---|---|---|---|
| H2 | 76.2 | — | 38.1 | 114.2 | 102.1 | 64.0 | — |
| CO | 75.7 | — | 36.7 | 112.5 | 98.4 | 61.7 | — |
| CH4 | — | — | 2.1 | 2.1 | 5.6 | 3.5 | — |
| C2H6 | — | — | 0.2 | 0.2 | 0.4 | 0.3 | — |
| CO2 | — | — | 3.6 | 3.6 | 9.7 | 6.1 | — |
| CH3OH | — | 12.6 | — | 12.6 | 11.1 | — | 11.1 |
| C2H5OH | — | — | — | — | 2.1 | — | 2.1 |
| C3H7OH | — | — | — | — | 0.4 | — | 0.4 |
| H2O | — | — | — | — | 0.6 | — | 0.6 |
| Totals | 151.9 | 12.6 | 80.7 | 245.2 | 230.4 | 135.5 | 14.2 |

|   |   |
|---|---|
| 1+2+3 | 245.2 |
| 6+7+8 | 230.4 |

0 253 540

Table 5 Product Yields and Compositions with Methanol Recycle

| Example | 1 | 2 | 3 | 4 | 5 |
|---|---|---|---|---|---|
| Yields(g/h/kg catalyst) | | | | | |
| CH3OH | +140.1 | +66.4 | -41.2 | -48.6 | |
| C2H5OH | 33.6 | 82.0 | 100.5 | 97.6 | |
| C3H7OH* | 9.3 | 21.8 | 28.0 | 25.4 | |
| H2O | 3.7 | 10.6 | 20.9 | 11.2 | |
| | | | | | |
| CO2 | 96.9 | 237.0 | 165.5 | 268.4 | |
| CH4/C2H6 | 19.5 | 51.2 | 33.2 | 54.5 | |
| | | | | | |
| Net import of CH3OH | | | | | |
| (g/h/kg catalyst) | 0.0 | 0.0 | 41.2 | 48.6 | |
| Selectivity(%) | | | | | |
| (H2O/CO2 free) | | | | | |
| CH4/C2H6 | 9.6 | 23.1 | 20.5 | 34.4 | |
| CH3OH | 69.2 | 30.0 | 0.0 | 0.0 | |
| C2H5OH | 16.6 | 37.0 | 62.2 | 52.1 | |
| C3H7OH* | 4.6 | 9.8 | 17.3 | 13.6 | |
| Composition | | | | | |
| (wt % dry) | | | | | |
| CH3OH | 76.6 | 39.0 | 0.0 | 0.0 | |
| C2H5OH | 18.4 | 48.2 | 78.2 | 79.3 | |
| C3H7OH* | 5.1 | 12.8 | 21.8 | 20.7 | |
| Alcohol yields (dry) | | | | | |
| (g/h/kg catalyst) | 183.0 | 170.0 | 129.0 | 123.0 | |

* includes some butanol

Table 6

| Example | 6 | 7 |
|---|---|---|
| Operating conditions | | |
|   Temperature (*C) | 300 | 300 |
|   Pressure (bar) | 100 | 100 |
|   GHSV (nl/h/kg cat) | 3200 | 5300 |
|   H2:CO | 2 | 2 |
| Conversions(/h/kg cat) | | |
|   CO(g) | 1097 | 1310 |
|   CO(wt% of CO fed) | 46 | 34 |
| Yields(g/h/kg cat) | | |
|   CH3OH | 242 | 452 |
|   C2H5OH | 152 | 176 |
|   C3H7OH* | 58 | 54 |
|   H2O | 47 | 29 |
|   CO2 | 563 | 594 |
|   CH4/C2H6 | 144 | 151 |
| Without methanol recycle | | |
| Alcohols(dry g/h/kg cat) | 452 | 682 |
|   CH3OH(%) | 54 | 66 |
|   C2H5OH(%) | 33 | 26 |
|   C3H7OH(%)* | 13 | 8 |
| With methanol recycle | | |
| Alcohols(dry g/h/kg cat) | 452 | 682 |
|   CH3OH(%) | 0 | 0 |
|   C2H5OH(%) | 80 | 80 |
|   C3H7OH(%)* | 20 | 20 |

* includes some butanol

## Claims

1. A process for the production of ethanol, propanol and butanol with substantially no net production of methanol, characterised in that methanol and synthesis gas is passed over an alcohol synthesis catalyst under alcohol synthesis conditions, and the product of alcohol synthesis is fractionated to yield a fraction containing ethanol, propanol and butanol and a fraction containing methanol and said fraction containing methanol is recycled, whereby there is substantially no net production of methanol.

2. A process according to claim 1, characterised in that the alcohol synthesis catalyst comprises $MoS_2$ promoted with $K_2CO_3$ and supported on active carbon.

3. A process according to claim 2, characterised in that the catalyst is prepared according to UK Patent Application No.2,185,907A.

4. A process according to any one of the preceding claims, characterised in that the synthesis reaction temperature is 270 to 300°C..

5. A process according to any one of the preceding claims, characterised in that the synthesis reaction pressure is 100 to 160 bar.

6. A process according to any one of the preceding claims, characterised in that a Gas Hourly Space Velocity of 2,000 to 20,000 $h^{-1}$ is used.

7. A process according to any one of the preceding claims, characterised in that the volume ratio of $H_2$:CO is approximately 1.2:1.

8. A process for the production of mixed alcohols comprising a desired small quantity of methanol, characterised in that a process according to any one of the preceding claims is modified by recycling a proportion of the methanol in the product of the alcohol synthesis and incorporating the remaining proportion in a mixed alcohol product containing the desired small quantity of methanol.

9. A process according to any one of the preceding claims, characterised in that methanol is imported to the process to supplement recycled methanol.

10. A process according to any one of the preceding claims, characterised in that the methanol to carbon monoxide volume ratio is from 1:5 to 1:0.

11. A process according to any one of the preceding claims, characterised in that the methanol to hydrogen volume ratio is from 1:0 to 1:1.

12. A mixed alcohol product produced by a process according to any one of the preceding claims.

13. The use of the mixed alcohol product of claim 12, as an octane improver in gasoline.

FIG. 1

0 253 540

FIG. 2

0 253 540

European Patent Office

. Application number

| | DOCUMENTS CONSIDERED TO BE RELEVANT | | EP 87305858.0 |
|---|---|---|---|

| Category | Citation of document with indication, where appropriate, of relevant passages | Relevant to claim | CLASSIFICATION OF THE APPLICATION (Int. Cl.4) |
|---|---|---|---|
| X | DE - A1 - 3 012 900 (INSTITUT FRANCAIS DU PETROLE)<br>* Claims 1,5 *<br>-- | 1,8 | C 07 C 29/15<br>C 07 C 29/32<br>C 07 C 31/02<br>C 10 L 1/10<br>B 01 J 27/051 |
| X | DE - A2 - 180 719 (THE DOW CHEMICAL)<br>* Abstract; claims 1,4-12 *<br>-- | 1-13 | |
| D,A | US - A - 3 972 952 (ROGER T. CLARK)<br>* Abstract *<br>-- | 1 | |
| D,A | EP - A1 - 0 013 464 (GULF RESEARCH)<br>* Abstract *<br>-- | 1 | |
| D,A | EP - A2 - 0 084 833 (RUHRCHEMIE)<br>* Abstract *<br>-- | 1 | TECHNICAL FIELDS SEARCHED (Int. Cl.4)<br><br>C 07 C 29/00<br>C 07 C 31/00<br>B 01 J |
| D,A | EP - A1 - 0 119 609 (THE DOW CHEMICAL)<br>* Abstract; claims 1-5; page 21, lines 5-24; page 17, lines 2-7 *<br>-- | 1,2,4-7,10,11,13 | |
| D,A | EP - A2 - 0 149 255 (UNION CARBIDE)<br>* Claims 1-5 *<br>-- | 1,2,4,7 | |

The present search report has been drawn up for all claims

| Place of search | Date of completion of the search | Examiner |
|---|---|---|
| VIENNA | 10-09-1987 | REIF |

**European Patent Office**

| DOCUMENTS CONSIDERED TO BE RELEVANT | | | EP 87305858.0 | |
|---|---|---|---|---|
| Category | Citation of document with indication, where appropriate, of relevant passages | Relevant to claim | CLASSIFICATION OF THE APPLICATION (Int. Cl.4) | |
| D,L | GB - A - 2 185 907 (COAL INDUSTRY) <br> * Abstract; claims 1,7,12,19 * <br> -- | 1-4,6, 13 | |
| A | EP - A1 - 0 167 300 (HUMPHREYS & GLASGOW) <br> * Abstract * <br> -- | 1,13 | |
| A | EP - A2 - 0 170 973 (THE DOW CHEMICAL) <br> * Abstract * <br> -- | 1 | |
| P,A | US - A - 4 607 056 (MICHAEL V. GRAZIOSO et al.) <br> * Abstract * <br> -- | 1 | TECHNICAL FIELDS SEARCHED (Int. Cl.4) |
| A | DE - A1 - 3 242 697 (RUHRCHEMIE) <br> * Claims 1-3 * <br> ----. | 1,8 | |

The present search report has been drawn up for all claims

| Place of search | Date of completion of the search | Examiner |
|---|---|---|
| VIENNA | 10-09-1987 | REIF |